# EUROPÄISCHE PATENTSCHRIFT

(11) **EP 1 267 854 B1**
(45) Veröffentlichungstag und Bekanntmachung des Hinweises auf die Patenterteilung: **21.11.2007**
(21) Anmeldenummer: 01938022.9
(22) Anmeldetag: 15.03.2001
(51) Int. Cl.: A61K 31/00

(54) **HOCHSELEKTIVE INHIBITOREN DES UROKINASE-PLASMINOGENAKTIVATORS**
HIGHLY SELECTIVE INHIBITORS OF THE UROKINASE PLASMINOGEN ACTIVATOR
INHIBITEURS A SELECTIVITE ELEVEE DE L'ACTIVATEUR DE TYPE UROKINASE DU PLASMINOGENE

(30) Priorität: 20.03.2000 DE 10013715
(43) Veröffentlichungstag der Anmeldung: 02.01.2003
(73) Patentinhaber: Wilex AG, 81675 München (DE); Max-Planck-Gesellschaft zur Förderung der Wissenschaften e.V., 80539 München (DE)
(72) Erfinder: MAGDOLEN, Viktor, 85551 Kirchheim (DE); SPERL, Stefan, 82049 Pullach (DE); STÜRZEBECHER, Jörg, 99094 Erfurt (DE); WILHELM, Olaf, 81545 München (DE); ARROYO-DEPRADA, Nuria, E-08013 Barcelona (ES); MORODER, Luis, 82152 Martinsried (DE); HUBER, Robert, 82110 Germering (DE); JACOB, Uwe, 80339 München (DE); BODE, Wolfram, 82131 Gauting (DE)
(74) Vertreter: Weiss, Wolfgang
(86) Internationale Anmeldenummer: PCT/EP2001/002989
(87) Internationale Veröffentlichungsnummer: WO 2001/070204

(56) Entgegenhaltungen:
- WO-A-00/04954
- WO-A-01/14324
- WO-A-99/20608
- US-A- 5 914 319
- SPERL S ET AL: "(4-Aminomethyl)phenylguanidine derivatives as nonpeptidic highly selective inhibitors of human urokinase" PROCEEDINGS OF THE NATIONAL ACADEMY OF SCIENCES OF USA, NATIONAL ACADEMY OF SCIENCE. WASHINGTON, US, Bd. 97, Nr. 10, 9. Mai 2000 (2000-05-09), Seiten 5113-5118, XP002169711 ISSN: 0027-8424
- RAI R ET AL: "GUANIDINOPHENYL-SUBSTITUTED ENOL LACTONES AS SELECTIVE MECHANISM-BASED INHIBITORS OF TRYPSIN-LIKE SERINE PROTEASES" JOURNAL OF MEDICINAL CHEMISTRY, AMERICAN CHEMICAL SOCIETY. WASHINGTON, US, Bd. 35, Nr. 22, 1992, Seiten 4150-4159, XP000978771 ISSN: 0022-2623
- HEECHUNG YANG ET AL: "SELECTIVE INHIBITION OF UROKINASE BY SUBSTITUTED PHENYLGUANIDINES: QUANTITATIVE STRUCTURE-ACTIVITY RELATIONSHIP ANALYSES" JOURNAL OF MEDICINAL CHEMISTRY, AMERICAN CHEMICAL SOCIETY. WASHINGTON, US, Bd. 33, Nr. 11, 1990, Seiten 2956-2961, XP002059264 ISSN: 0022-2623

## Beschreibung

Die vorliegende Erfindung betrifft neue hochselektive Inhibitoren des Urokinase-Plasminogenaktivators (uPA, EC 3.4.21.31) vom Arylguanidintyp.

Der Plasminogenaktivator von Urokinase-Typ (uPA) spielt eine Schlüsselrolle bei der Tumorinvasion und Metastasenbildung (Schmitt et al., J. Obst. Gyn. 21 (1995), 151-165). uPA wird in verschiedenen Arten von Tumorzellen überexprimiert (Kwaan, Cancer Metastasis Rev. 11 (1992), 291-311) und bindet an den Tumor-assoziierten uPA-Rezeptor (uPA-R), wo die Aktivierung von Plasminogen zu Plasmin stattfindet. Plasmin ist in der Lage, verschiedene Komponenten der extrazellulären Matrix (ECM) wie Fibronectin, Laminin und Kollagen Typ IV abzubauen. Es aktiviert auch einige andere ECM-abbauende Enzyme, insbesondere Matrix-Metalloproteinasen. Hohe Mengen an Tumor-assoziiertem uPA korrelieren mit einem höheren Metastasierungsrisiko für Krebspatienten (Stephens et al., Breast Cancer Res. & Treat. 52 (1998), 99-111). Eine Hemmung der proteolytischen Aktivität von uPA ist daher ein guter Ansatzpunkt für eine anti-metastatische Therapie.

Ein gemeinsames Merkmal vieler bekannter synthetischer uPA-Inhibitoren ist ein basischer Rest, der Amidino- oder Guanidino-Gruppen enthält, und an Asp¹⁸⁹ in der S1-Spezifitätstasche von uPA binden kann und dort als Arginin-Mimetikum wirkt (Spraggon et al., Structure 3 (1995), 681-691). Die meisten der bekannten Inhibitoren sind jedoch nicht selektiv für uPA, sondern hemmen auch andere Serinproteasen wie Trypsin, Thrombin, Plasmin oder Gewebs-Plasminogenaktivator (tPA).

p-Aminobenzamidin ist ein moderat selektiver uPA-Inhibitor mit einer Hemmkonstante von 82 µM. Billstroem et al. (Int. J. Cancer 61 (1995), 542-547) konnten eine deutliche Abnahme der Wachstumsrate von DU 145 Tumoren (eine Prostata-Adenokarzinom-Zellinie) in SCID Mäusen bei oraler Verabreichung in einer Tagesdosis von 125 bis 250 mg p-Aminobenzamidin/kg/Tag zeigen. Die Nebenwirkungen waren vernachlässigbar gering.

Einige monosubstituierte Phenylguanidine haben sich als wirksame und selektive uPA Inhibitoren in vitro erwiesen. Diese kleinen Moleküle zeigen Inhibierungskonstanten im Mikromolarbereich, sie binden jedoch nur in der S1 Tasche von uPA (Yang et al., J. Med. Chem. 33 (1990), 2956-2961). Biologische Untersuchungen mit diesen Verbindungen wurden nicht durchgeführt.

Das Diuretikum Amilorid ist ein selektiver uPA-Inhibitor (Ki, uPA = 7 µM), der die Bildung von Lungenmetastasen nach i.v. Inokulation von Ratten-Brustadenokarzinomzellen verhindert (Kellen et al., Anticancer Res. 8 (1988), 1373-1376). Einige Derivate von 3-Amidino-phenylalanin haben sich ebenfalls als wirksame Inhibitoren von Serinproteasen erwiesen, diese Verbindungen weisen jedoch im allgemeinen nur eine geringe Selektivität für uPA auf (Stürzebecher et al., J. Med. Chem. 40 (1997), 3091-3099; Stürzebecher et al., J. Enzyme Inhib. 9 (1995), 87-99).

Die derzeit wirksamsten und selektivsten uPA-Inhibitoren sind Derivate von Benzo[b]thiophen-2-carboxamidin (B428 und B623: Kᵢ, uPA = 0,32 bzw. 0,07 µM; US-Patent 5,340,833). Rabbani et al. (Int. J. Cancer 63 (1995), 840-845) sowie Xing et al. (Cancer Res. 57 (1997), 3585-3593) konnten nach Verabreichung von 4-lod-benzo[b]-thiophen-2-carboxamidin (B428) eine Abnahme des Tumorwachstums und der Metastasenbildung in einem syngenen Modell für Ratten-Prostatakarzinom bzw. Maus-Mammakarzinom zeigen. Letztere Untersuchungen zeigten eine weitere Abnahme des Primärtumorwachstums bei gemeinsamer Verabreichung von B428 mit dem Antiöstrogen Tamoxifen.

Die deutsche Patentanmeldung 199 40 389.9 schlägt die Verwendung von Arylguanidin- und insbesondere Phenylguanidin-Derivaten als selektive uPA-Inhibitoren vor. Diese Verbindungen enthalten einen weiteren Substituenten am aromatischen Ringsystem, vorzugsweise in Para-Position zur Guanidingruppe, der eine gegebenenfalls substituierte Methylengruppe gefolgt von Wasserstoffdonor/Akzeptorfunktionalitäten enthält. Aufgrund dieses Substitutionsmusters weisen die Verbindungen eine besonders hohe Wirksamkeit und Selektivität für uPA auf. Von diesen Verbindungen wird angenommen, dass sie als Arginin-Mimetikum mit dem Aminosäurerest Asp¹⁸⁹ in der S1 -Tasche von uPA wechselwirken und eine Wechselwirkung mit der S2- und/oder S3-Tasche von uPA eingehen können.

In der internationalen Patentanmeldung WO 01/14324 werden Derivate des 3-Amidino-phenylalanins, insbesondere Guanidinium-Verbindungen offenbart, welche als Urokinase-Inhibitoren zur Behandlung von Urokinase- oder Urokinase-Rezeptor-assoziierten Krankheiten verwendet werden.

Alternativ werden in der internationalen Patentanmeldung WO 99/20608 Isoquinoline, insbesondere Isoquinolin-Guaninidine als Urokinase-Inhibitoren vorgeschlagen.

Yang et al. berichten in J. Med. Chem. 1990, 33, 2956-2961 von der Verwendung von substituierten Phenylguanidinen zur selektiven Inhibierung von Urokinase.

Überraschenderweise wurden nun weitere Aryl-Guanidin-Derivate identifiziert, die eine noch spezifischere Wechselwirkung mit uPA, insbesondere mit den Aminosäureresten Gln¹⁹² und/oder Ser²¹⁴ eingehen können. Diese Verbindungen enthalten neben der Guanidin-Gruppe einen weiteren Substituenten am aromatischen Ringsystem, der eine gegebenenfalls substituierte Methylengruppe gefolgt von einer Wasserstoffdonor-, einer Wasserstoffakzeptor- und wiederum einer Wasserstoffdonorfunktionalität enthält.

Ein Gegenstand der vorliegenden Erfindung ist die Verwendung von Verbindungen der allgemeinen Formel (I) 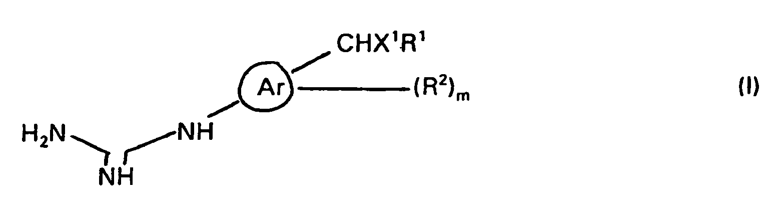 worin
- Ar: ein aromatisches oder heteroaromatisches Ringsystem bedeutet,
- X¹: einen Rest der Formel (IIa), (IIb) oder (IIc) bedeutet:

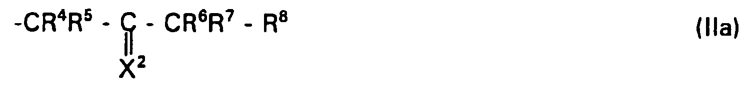

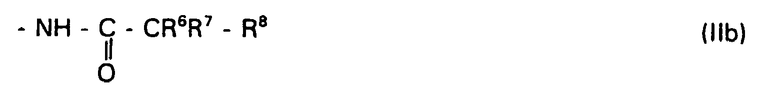

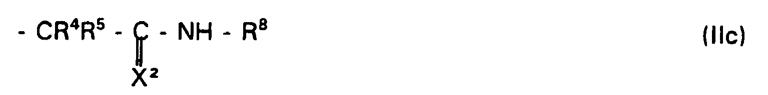

- R¹: H, einen gegebenenfalls substituierten Alkyl-, Alkenyl-, Alkinyl-, Aryl- oder/und Heteroarylrest bedeutet,
- R²: Halogen, C(R³)₃), C₂(R³)₅, OC(R³)₃ oder OC₂(R³)₅ bedeutet,
- R³: jeweils unabhängig H oder Halogen, insbesondere F, ist
- R⁴ und R⁵: H oder einen gegebenenfalls substituierten Alkyl-, Alkenyl- oder Alkinylrest bedeuten, wobei zumindest einer der Reste R⁴ und R⁵ eine Wasserstoffbrücken-Donorgruppe, z.B. OH, NH₂, SH, OR¹, NHR¹, N(R¹)₂, SR¹, CO, CS, enthält,
- R⁶ und R⁷: H oder einen gegebenenfalls substituierten Alkyl-, Alkenyl- oder Alkinylrest bedeuten, wobei zumindest einer der Reste R⁶ und R⁷ eine Wasserstoffbrücken-Donorgruppe, z.B. OH, NH₂, SH, OR¹, NHR¹, N(R¹)₂, SR¹,CO, CS, enthält, und wobei R⁴ oder R⁵ mit R⁶ oder R⁷ verbrückt sein kann,
- R⁸: H oder einen gegebenenfalls substituierten Alkyl-, Alkenyl-, Alkinyl-, Aryl- oder/und Heteroarylrest oder -SO₂-R⁹ bedeutet, wobei R⁸ gegebenenefalls mit R⁶ oder R⁷ verbrückt sein kann,
- R⁹: H oder einen gegebenenfalls substituierten Alkyl-, Alkenyl-, Alkinyl-, Aryl- oder/und Heteroarylrest bedeutet,
- X²: eine Wasserstoffbrücken-Akzeptorgruppe, insbesondere NH, NR¹⁰, O oder S bedeutet,
- R¹⁰: einen gegebenenfalls substituierten Alkyl-, Alkenyl- oder Alkinylrest bedeutet, und
- m: eine ganze Zahl von 0 bis 4 ist,
oder Salzen dieser Verbindungen zur Herstellung eines Mittels zur Hemmung des Urokinase-Plasminogenaktivators.

Die Verbindungen können als Salze, vorzugsweise als physiologisch verträgliche Säuresalze, z.B. als Salze von Mineralsäuren, besonders bevorzugt als Hydrochloride oder als Salze von geeigneten organischen Säuren vorliegen. Die Guanidiniumgruppe kann gegebenenfalls Schutzfunktionen tragen, die vorzugsweise unter physiologischen Bedingungen abspaltbar sind. Die Verbindungen können als optisch reine Verbindungen oder als Gemische von Enantiomeren oder/und Diastereoisomeren vorliegen.

In den Verbindungen der allgemeinen Formel (I) ist Ar vorzugsweise ein aromatisches oder heteroaromatisches Ringsystem mit einem einzigen Ring, insbesondere ein Benzolring. In diesem Ringsystem sind die Substituenten CHX¹R¹ und NHC(NH)NH₂ vorzugsweise in Meta- oder Para-Position und besonders bevorzugt in Para-Position zueinander angeordnet. Darüber hinaus kann Ar noch weitere von Wasserstoff verschiedene Substituenten R² enthalten. Vorzugsweise ist die Anzahl der Substituenten R² 0, 1, 2 oder 3, besonders bevorzugt 0 oder 1 und am meisten bevorzugt O. Bevorzugte Beispiele für R² sind Halogenatome (F, Cl, Br oder I), CH₃, CF₃, OH, OCH₃ oder OCF₃.

Für die Inhibitoraktivität kritisch ist der Substituent -CHX¹R¹. R¹ kann H oder ein gegebenenfalls substituierter Alkyl-, Alkenyl-, Alkinyl-, Aryloder/und Heteroarylrest sein. Der Alkylrest kann eine geradkettige oder verzweigte C₁-C₁₀-Alkylgruppe, insbesondere eine C₁-C₄-Alkylgruppe, oder eine C₃-C₈-Cycloalkylgruppe sein, die beispielsweise mit C₁-C₃-Alkoxy, Hydroxyl, Carboxyl, Amino, Sulfonyl, Nitro, Cyano, Oxo oder/und Halogen, aber auch mit Aryl- oder Heteroarylresten substituiert sein kann. Alkenyl- und Alkinylreste sind vorzugsweise C₂-C₁₀-Gruppen, insbesondere C₂-C₄-Gruppen, die gegebenenfalls wie zuvor angegeben substituiert sein können. Aryl- und Heteroarylreste können beispielsweise mit C₁-C₆-Alkyl, C₁-C₃-Alkoxy, Hydroxyl, Carboxyl, Sulfonyl, Nitro, Cyano oder/und Oxo substituiert sein.

Die Gruppe X¹ enthält vorzugsweise zumindest einen oder zwei Substituenten (R⁴ oder R⁵ oder/und R⁶ oder R⁷), die eine Wasserstoffbrücken-Donorgruppe enthalten, sowie einen Substituenten X², der eine Wasserstoffbrücken-Akzeptorgruppe enthält. Die Wasserstoffbrücken-Donorsubstituenten enthalten eine ein Wasserstoffatom oder/und ein Elektronenpaar für eine Wasserstoffbrücke liefernde Gruppe. Der Abstand der Wasserstoffbrücken-Donorgruppe vom C-Atom, an das die Substituenten R⁴ und R⁵ bzw. R⁶ und R⁷ gebunden sind, beträgt vorzugsweise 1 bis 3 Kohlenstoffatome, besonders bevorzugt 1 bis 2 Kohlenstoffatome und am meisten bevorzugt 1 Kohlenstoffatom. Beispiele für Wasserstoffbrücken-Donorgruppen sind OH, NH₂, SH, OR¹, NHR¹, N(R¹)₂, SR¹. Bevorzugte Beispiele für Wasserstoffbrücken-Donorgruppen enthaltende Substituenten sind Hydroxymethyl, 2-Hydroxyethyl, -CO₂H, -CO₂R¹, wobei R¹ wie zuvor definiert ist und vorzugsweise eine Alkyl- oder eine Arylgruppe wie etwa die Benzylgruppe bedeutet, z.B. -CO₂CH₂-Ph, CONH₂, -CONHR¹, -CONR¹, wobei R¹ wie zuvor definiert ist, z.B. CONHCH₃, -CON(CH₃)₂, CSOH, CSOR¹, -COSH, COSR¹, COR¹ und CSR¹, wobei R1 wie zuvor definiert ist. Zwei Wasserstoffbrücken-Donorgruppen enthaltende Substituenten können auch verbrückt sein, z.B. über eine C₂-C₃-Brücke. Beispiele für solche verbrückten Substituenten sind 1,2-Dihydroxy-ethylen oder 1,3-Dihydroxypropylen.

Ein Substituent R⁴/R⁵ bzw. R⁶/R⁷, der keine Wasserstoffbrücken-Donorgruppe trägt, ist vorzugsweise Wasserstoff oder eine gegebenenfalls Halogensubstituierte Methyl- oder Ethylgruppe. Besonders bevorzugt ist ein solcher Substituent Wasserstoff.

Der Wasserstoffbrücken-Akzeptorsubstituent X² ist vorzugsweise NH oder O, besonders bevorzugt O.

Weiterhin enthält die Gruppe X¹ einen Substituenten R⁸, der vorzugsweise eine sterische Funktion erfüllt. R⁸ kann Wasserstoff, ein Alkyl-, Alkenyl-, Alkinyl-, Aryl-, Heteroaryl-, Carboxy-alkyl-, Carboxy-alkenyl-, Carboxy-alkinyl-, Carboxy-aryl- oder Carboxy-heteroaryl-Rest oder -SO₂-R⁹ sein, wobei R⁹ die gleiche Bedeutung wie für R⁸ angegeben besitzen kann. Günstigerweise sind R⁸ bzw. R⁹ von Wasserstoff verschieden und enthalten mindestens 4, z.B. 6 bis 20 Kohlenstoffatome. R⁸ kann gegebenenfalls mit R⁶ oder R⁷ verbrückt sein.

Die Substituenten R⁸ und R⁹ enthalten vorzugsweise raumfüllende Gruppen, die ausgewählt sein können aus gegebenenfalls substituierten Arylresten, insbesondere Phenyl- und substituierten Phenylresten und gegebenenfalls substituierten verzweigte Alkyl-, Alkenyl- oder Alkinylresten, insbesondere mit tertiären C-Atomen wie tert.-Butyl oder Neopentyl oder gegebenenfalls substituierten Cycloalkylresten, insbesondere Bi- oder Tricycloalkylresten wie Adamantyl.

Die Verbindungen der allgemeinen Formel (I) können beispielsweise ausgehend von p-Amino-benzylamin gemäß den in der deutschen Patentanmeldung 199 40 389.9 gezeigten Reaktionsschemata hergestellt werden. 4-Amino-benzylamin kann beispielsweise mit einem Schutzreagenz für Aminogruppen, z,B, Di-tert-butyl-pyrocarbonat zu einem geschützten Zwischenprodukt 4-(N-Boc-Aminomethyl)-anilin umgesetzt werden, wobei Boc tert-Butyloxycarbonyl bedeutet. Die aromatische Aminofunktion dieser Verbindung kann mit einem Guanidinylierungsreagenz, z.B. N,N'-Di-Z-N"-triflylguanidin umgesetzt werden, wobei 1-[4-(N-Boc-aminomethyl)-phenyl]-2,3-di-Z-guanidin entsteht, wobei Z Benzyloxycarbonyl bedeutet. Diese Verbindung kann durch Abspaltung der Boc-Schutzgruppe zu 1-[4-(Aminomethyl)-phenyl)-2,3-di-Z-guanidinium-hydrochlorid umgesetzt werden. Diese Verbindung kann wiederum mit reaktiven Verbindungen wie etwa Chlorameisensäureestern, Isocyanaten oder N-Hydroxysuccinimidestern zu den gewünschten Endprodukten umgesetzt werden.

Zur Darstellung hydrierungslabiler Verbindungen kann 4-Amino-benzylamin mit einem Schutzreagenz für Aminogruppen, z.B. Benzyloxycarbonyloxysuccinimid zu einem geschützten Zwischenprodukt und dann mit einem weiteren Guanidinylierungsreagenz, z.B. N,N'-Di-Boc-1-guanylpyrazol umgesetzt werden. Diese Verbindung kann hydriert und anschließend mit reaktiven Verbindungen zu den gewünschten Endprodukten umgesetzt werden.

Die erfindungsgemäßen Urokinaseinhibitoren können gegebenenfalls zusammen mit geeigneten pharmazeutischen Hilfs- oder Trägerstoffen zur Herstellung von Arzneimitteln oder in der Diagnostik verwendet werden. Dabei ist eine Verabreichung in Kombination mit anderen Wirkstoffen, z.B. anderen Urokinaseinhibitoren wie etwa Antikörpern oder/und Peptiden möglich.

Die Arzneimittel können bei Menschen und Tieren topisch, oral, rektal oder parenteral, z.B. subkutan oder intravenös, z.B. in Form von Tabletten, Dragees, Kapseln, Pellets, Suppositorien, Lösungen oder transdermalen Systemen, wie Pflastern, verabreicht werden.

Die erfindungsgemäßen Verbindungen sind zur Bekämpfung von Krankheiten geeignet, die mit einer pathologischen Überexpression von uPA oder/und uPAR assoziiert sind. Sie sind beispielsweise in der Lage, hocheffizient das Wachstum oder/und die Ausbreitung der malignen Tumoren sowie die Metastasierung von Tumoren zu hemmen. Dabei können die uPA-Inhibitoren gegebenenfalls zusammen mit anderen Tumormitteln oder mit anderen Behandlungsarten, z.B. Bestrahlung oder chirurgischen Eingriffen, eingesetzt werden. Weiterhin sind die erfindungsgemäßen Inhibitoren auch für andere uPA-assoziierte Erkrankungen wirksam.

Erfindungsgemäße uPA-Inhibitoren sind vorzugsweise dadurch gekennzeichnet, daß sie mindestens einen zweifach, vorzugsweise mindestens einen fünffach und besonders bevorzugt einen mindestens 10- und bis zu 1.000-fach geringeren Kᵢ-Wert für uPA gegenüber tPA aufweisen. Weiterhin ist bemerkenswert, dass die erfindungsgemäßen Verbindungen die Blutgerinnung nur geringfügig beeinflussen, da sie für eine effektive Hemmung von Thrombin, Plasmin und Faktor Xa zu hohe K₁-Werte haben.

Die erfindungsgemäßen Substanzen der Formel (I) können in Form von Konjugaten mit physiologisch wirksamen Substanzen eingesetzt werden, z.B. mit Radiomarkierungen oder mit zytotoxischen Mitteln, z.B. Chemotherapeutika wie cis-Platin oder 5-Fluor-uracil, oder Peptiden. Weiterhin können die Substanzen auch in die Membran von Trägervesikeln, z.B. Liposomen, eingebaut werden und somit ein Targeting von in den Trägervesikeln eingeschlossenen Wirksubstanzen, z.B. zytotoxischen Mitteln, wie etwa Doxorubicin, ermöglichen.

Durch die Erfindung wird ein Verfahren zur Urokinasehemmung bei Lebewesen, insbesondere bei Menschen, durch Verabreichung einer wirksamen Menge mindestens einer Verbindung der allgemeinen Formel (1) bereitgestellt. Die Dosierung der Verbindung liegt üblicherweise im Bereich von 0,01 bis 100 mg/kg Körpergewicht pro Tag. Die Dauer der Behandlung hängt von der Schwere der Erkrankung ab und kann von einer einmaligen Gabe bis zu einer mehrwöchigen oder sogar mehrmonatigen Behandlung, die gegebenenfalls in Intervallen wiederholt werden kann, reichen.

Schließlich betrifft die Erfindung neue Aryl-Guanidinderivate der allgemeinen Formel (I).

Die Erfindung soll durch die folgenden Beispiele und Abbildungen näher erläutert werden. Es zeigt:
- Figur 1: Beispiele für 5 erfindungsgemäße Verbindungen der Formel (I) und
- Figur 2: Beispiele für weitere bevorzugte Verbindungsklassen der Formel (I).

### Beispiele

### Material und Methoden

Alle für die Synthese von uPA-Inhibitoren verwendeten Lösungsmittel und Reagenzien waren von der höchsten kommerziell verfügbaren Qualität und wurden - sofern erforderlich - durch Standardmethoden weiter aufgereinigt und getrocknet. Die analytische HPLC erfolgte auf Nucleosil 100/C18 Säulen (Macherey-Nagel, Düren, Deutschland) unter Verwendung eines linearen Acetonitril/2% H₃PO₄ Gradienten (von 5:95 bis 90:10 in 13 min) MS-Spektren wurden auf einem Perkin Elmer API 165 Massenspektrometer gemessen.

### Beispiel 1

### Synthese von Verbindungen der Formel (I)

### ST390: D,L-Tropasäure-(4-Guanidinobenzyl)-amid Hydrochlorid.

Eine Lösung von 1-[4-Aminomethyl)-phenyl]-2,3-di-Z-guanidin-hydrochlorid (30 mg; 0,064 mmol), D,L-Tropasäure (10,6 mg; 0,064 mmol), 1-Hydroxybenzotriazol (HOBt) (10 mg; 0,07 mmol) und Triethylamin (10 µl; 0,192 mmol) in Dichlormethan (3 ml) wurde mit 2-(1H-Benzotriazol-1-yl)-1,1,3,3-tetramethyluronium-tetrafluoroborat (TBTU) (23 mg; 0,07 mmol) versetzt und bei Raumtemperatur gerührt. Nach 2 h wurden nochmals TBTU (15 mg; 0,047 mmol) und Triethylamin (10 µl; 0,192 mmol) zugegeben. Nach weiteren 3 h wurde mit 30 ml Dichlormethan verdünnt, 3 x mit 5% NaHCO₃-Lösung, 2 x mit 0,1 HCl und 1 x mit gesättigter NaCl-Lösung gewaschen. Die organische Phase wurde getrocknet (Na₂SO₄) und das Lösungsmittel im Vakuum abgezogen. Zur Abspaltung der Z-Schutzgruppen (Benzyloxycarbonyl-) wird die Verbindung in Methanol gelöst, gerührt und für 3 h über einem 10% Palladium-Aktivkohle-Katalysator hydriert. Nach Entfernung des Katalysators durch Filtration wurde das Lösungsmittel im Vakuum abgedampft. Das Produkt wurde aus Isopropanol/Diisopropylether nach Zugabe von 1 Äquivalent HCl in Dioxan umkristallisiert.

| | |
|---|---|
| Ausbeute: | 13 mg (58%); HPLC: t_{R} 5,6 min; MS 313 (M + H)⁺, berechnet 312 für C₁₇H₂₀N₄O₂ |

### ST399: 2-(Benzyloxycarbonyl)-2-Phenyl-Essigsäure-[4-(2,3-Bis-Boc-Guanidino)-benzyl]-Amid

Eine Lösung von 1-[4-(aminomethyl)-phenyl]-2,3-di-tert-Butyloxycarbonylguanidin Hydrochlorid (8) (100 mg;0,249 mmol), 2-Phenylmalonsäuremonobenzylester (67,3 mg; 0,249 mmol), HOBt (37 mg; 0,274 mmol) und Triethylamin (104 µl; 0,747 mmol) in DMF (5 ml) wurde mit TBTU (88 mg; 0,274 mmol) versetzt und bei Raumtemperatur über Nacht gerührt. Nach Abziehen des Lösungsmittels im Vakuum wurde der Rückstand in Ethylacetat (20 ml) aufgenommen, 3 x mit 5% NaHCO₃-Lösung, 3 x mit 0,5 M HCl und 1 x mit gesättigter NaCl-Lösung gewaschen. Die organische Phase wurde getrocknet (Na₂SO₄) und das Lösungsmittel im Vakuum abgezogen. Das Produkt wurde aus Ethanol/Wasser umkristallisiert.

| | |
|---|---|
| Ausbeute: | 78 mg (51 %); HPLC: t_{R} 13,0 min; MS 617 (M + H)⁺, berechnet 616 für C₃₄H₄₀N₄O₇ |

### ST401: 2-(Benzyloxycarbonyl)-2-Phenyl-Essigsäure-(4-Guanidinobenzyl)-Amid Hydrochlorid

Die Abspaltung der Boc-Schutzgruppen erfolgte durch Lösen der Verbindung ST399 (12 mg; 19,5 µM) in 7 M HCl/Dioxan (2 ml). Nach 8 h wurde das Lösungsmittel im Vakuum abgezogen und das Produkt aus Isopropanol/Diisopropylether umkristallisiert.

| | |
|---|---|
| Ausbeute: | 4 mg (45,4%); HPLC: t_{R} 9,0 min; MS (M + H)⁺, berechnet 416 für C₂₄H₂₄N₄O3 |

### ST406: 2-(Hydroxycarbonyl)-2-Phenyl-Essigsäure-(4-Guanidinobenzyl)-Amid Hydrochlorid

Die Abspaltung der Benzylgruppe von Verbindung ST399 (150 mg; 0,244 mmol) erfolgte durch katalytische Hydrierung in einer Lösung aus Isopropanol (30 ml) uind Dichlormethan an einem Pd/Aktivkohle-Katalysator. Nach 5 h wurde der Katalysator abfiltriert und das LM abgezogen. Das Produkt wurde nach Behandlung mit Methyl-tertButyl-ether im Ultraschallbad als hellgelbes Pulver erhalten. Anschließend wurden die Boc-Schutzgruppen, wie für ST401 beschrieben, abgespalten und das Rohprodukt durch präparative HPLC gereinigt.

| | |
|---|---|
| Ausbeute: | 37 mg (42%); HPLC: t_{R} 4,6 min; MS 327 (M + H)⁺, berechnet 326 für C₁₇H₁₈N₄O₃. |

### Beispiel 2

### In vitro Hemmung von Urokinase durch ausgewählte Verbindungen der Formel (I)

Zur Bestimmung der uPA Inhibitoraktivität wurden 200 µl Tris-Puffer (0,05 mol/l, den Inhibitor enthaltend, 0,154 mol/l NaCl, 5 % Ethanol, pH 8,0), 25 µl Substrat (Pefachrome UK oder BZ-β-Ala-Gly-Arg-pNA in H₂O; Pentapharm LTD, Basel, Schweiz) und 50 µl sc-Urokinase (Ribosepharm GmbH, Haan, Deutschland) bzw. eine entsprechende andere Protease bei 25 °C inkubiert. Nach 3 min wurde die Reaktion durch Zugabe von 25 µl Essigsäure (50 %) unterbrochen und die Absorption bei 405 nm mittels eines Mikroplate Reader (MR 5000, Dynatech, Denkendorf, Deutschland) bestimmt. Die Kᵢ-Werte wurden nach Dixon durch lineare Regression mittels eines Computerprogramms ermittelt. Die Kᵢ-Werte sind das Mittel aus mindestens drei Bestimmungen, die Standardabweichung lag unter 25 %.

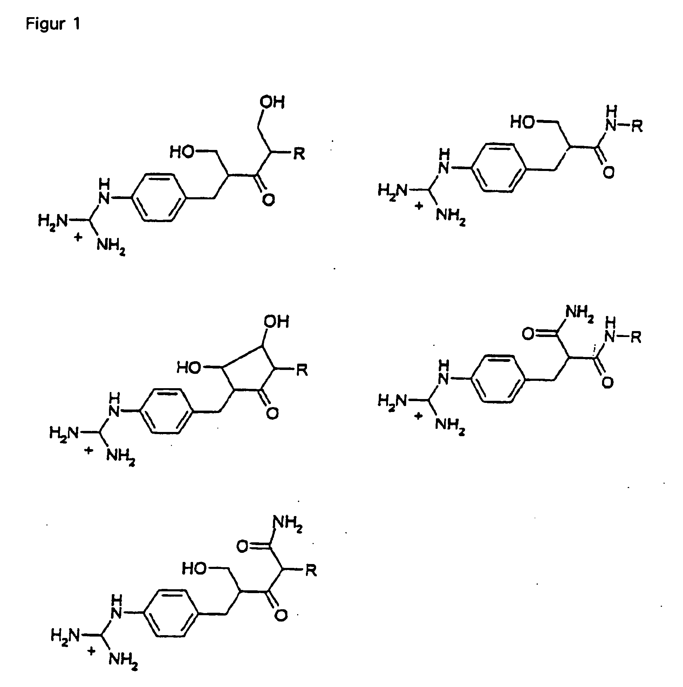

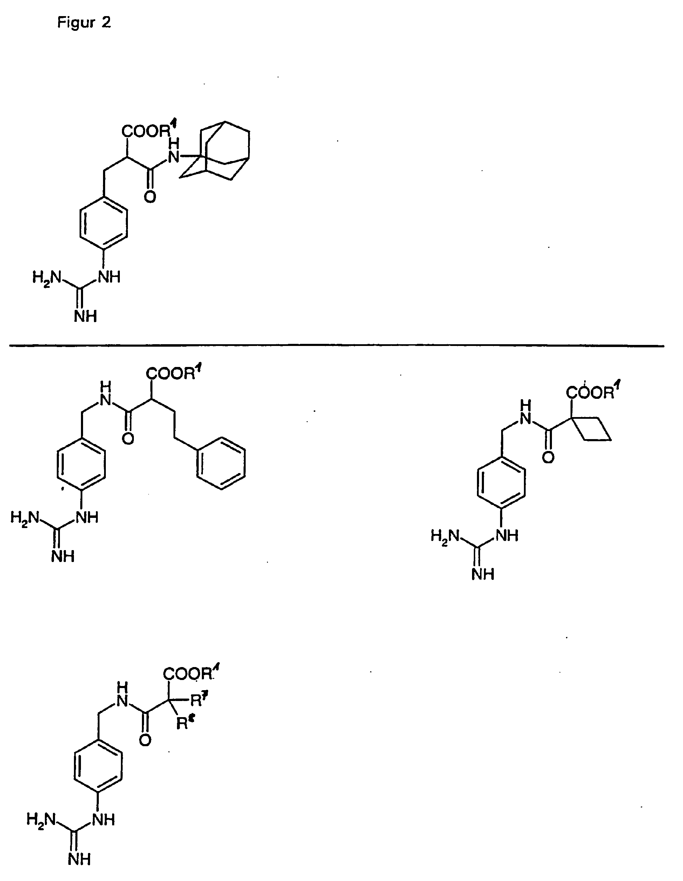

Die in Figur 1 und 2 gezeigten Verbindungen können aufgrund ihrer Struktur mit dem aktiven Zentrum von uPA wechselwirken. Die Ergebnisse für ausgewählte Verbindungen der Formel (I) sind in Tabelle 1 dargestellt.

**Tabelle 1**

| **Kᵢ[µM]** | | | | | | |
|---|---|---|---|---|---|---|
| Nr. | Formel | uPA | Plasmin | FXa | Thrombin | Trypsin |
| ST-390 | 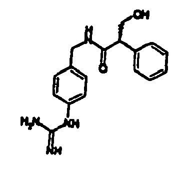 | 51 | >1000 | >1000 | >1000 | >1000 |
| ST-401 | 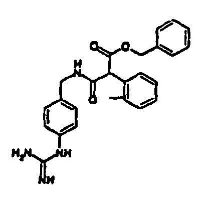 | 1,4 | >1000 | >1000 | >1000 | n.t. |
| S.T-406 | 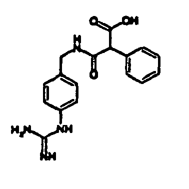 | <10 | n.t. | n.t. | n.t. | n.t. |
| ST-100 | 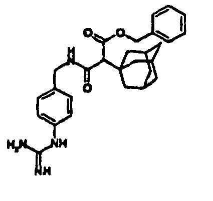 | < 1 | n.t. | n.t. | n.t. | n.t. |

| | | | | | | |
|---|---|---|---|---|---|---|
| n.t. = nicht getestet | | | | | | |

## Patentansprüche

1. Verwendung von Verbindungen der allgemeinen Formel (I) 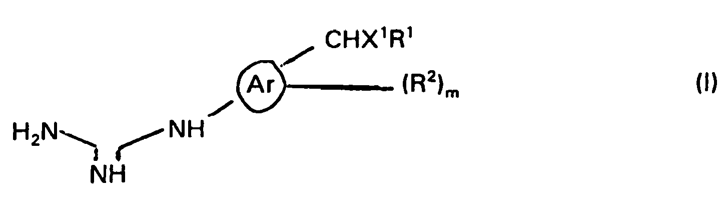 worin
Ar einen Benzolring bedeutet,
X¹ einen Rest der Formel (IIa), (IIb) oder (IIc) bedeutet: 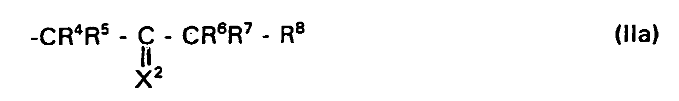 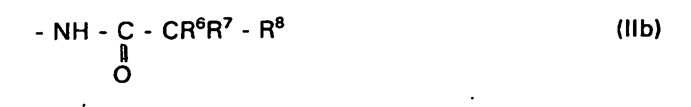 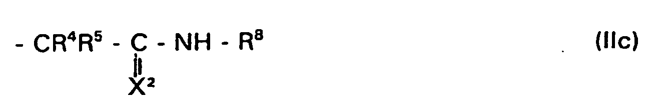
R¹ H, einen gegebenenfalls substituierten Alkyl-, Alkenyl-, Alkinyl-, Aryl- oder/und Heteroarylrest bedeutet,
R² Halogen, C(R³)₃), C₂(R³)₅, OC(R³)₃ oder OC₂(R³)₅ bedeutet,
R³ jeweils unabhängig H oder Halogen, insbesondere F, ist,
R⁴ und R⁵ H oder einen gegebenenfalls substituierten Alkyl-, Alkenyl- oder Alkinylrest bedeuten, wobei zumindest einer der Reste R⁴ und R⁵ eine Wasserstoffbrücken-Donorgruppe enthält, wobei die Wasserstoffbrücken-Donorgruppe ein H-Atom oder/und ein Elektronenpaar für eine Wasserstoffbrückenbindung liefert,
R⁶ und R⁷ H oder einen gegebenenfalls substituierten Alkyl-, Alkenyl oder Alkinylrest bedeuten, wobei zumindest einer der Reste R⁶ und R⁷ eine Wasserstoffbrücken-Donorgruppe enthält, wobei die Wasserstoffbrücken-Donorgruppe ein H-Atom oder/und ein Elektronenpaar für eine Wasserstoffbrückenbindung liefert, und wobei R⁴ oder R⁵ mit R⁶ oder R⁷ verbrückt sein kann,
R⁸ H oder einen gegebenenfalls substituierten Alkyl-, Alkenyl-, Alkinyl-, Aryl- oder/und Heteroarylrest oder -SO₂-R⁹ bedeutet, wobei R⁸ mit R⁶ oder R⁷ verbrückt sein kann,
R⁹ H oder einen gegebenenfalls substituierten Alkyl-, Alkenyl-, Alkinyl-, Aryl- oder/und Heteroarylrest bedeutet,
X² eine Wasserstoffbrücken-Akzeptorgruppe, insbesondere NH, NR¹⁰, O oder S, bedeutet,
R¹⁰ einen gegebenenfalls substituierten Alkyl-, Alkenyl- oder Alkinylrest bedeutet und
m eine ganze Zahl von 0 bis 4 ist,
oder Salzen dieser Verbindungen zur Herstellung eines Mittels zur Hemmung des Urokinase-Plasminogenaktivators.

2. Verwendung von Verbindungen nach Anspruch 1, worin die Substituenten -CHX¹R¹ und -NHC(NH)NH₂ in para-Position zueinander angeordnet sind.

3. Verwendung nach einem der Ansprüche 1 bis 2, worin bei R⁴, R⁵, R⁶ und R⁷ die Wasserstoffbrücken-Donorgruppen aus OH, OR¹, NH₂, NHR¹, N(R¹)₂, SH und SR¹ ausgewählt sind.

4. Verwendung nach Anspruch 3, worin die Wasserstoffbrücken-Donorgruppen enthaltenden Substanzen ausgewählt sind aus -CH₃OH, -CH₂CH₂OH, -CO₂H, -CO₂R¹, CONH₂, -CONHR¹, -CON(R¹¹)₂, -COSH, -COSR¹, -CSOH, COSR¹, COR¹ und CSR¹, wobei R¹ wie in Anspruch 1 definiert ist.

5. Verwendung nach einem der Ansprüche 1 bis 4, worin bei X² die Wasserstoffbrücken-Akzeptorgruppen aus O und NH ausgewählt sind.

6. Verwendung von Verbindungen nach einem der Ansprüche 1 bis 5, worin R⁸ und R⁹ ausgewählt sind aus gegebenenfalls substituierten Aryl-, insbesondere Phenyl- und substituierten Phenylresten, und gegebenenfalls substituierten tertiären Alkylresten oder Cycloalkylresten, insbesondere Bicycloalkylresten wie Adamantyl.

7. Verwendung nach einem der Ansprüche 1 bis 6 zur Bekämpfung von Krankheiten, die mit einer pathologischen Überexpression von Urokinase oder/und Urokinase-Rezeptor assoziiert sind.

8. Verwendung nach Anspruch 7 zur Tumorbekämpfung.

9. Verwendung nach Anspruch 7 oder 8 zur Bekämpfung der Metastasenbildung.

10. Verwendung nach einem der vorhergehenden Ansprüche zur Herstellung von oral, topisch, rektal oder parental verabreichbaren Arzneimitteln.

11. Verwendung nach einem der vorhergehenden Ansprüche in Form von Tabletten, Dragees, Kapseln, Pellets, Suppositorien, Lösungen oder transdermalen Systemen wie Pflastern.

12. Verbindungen der Formel (I) 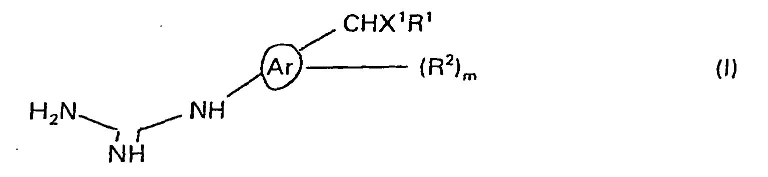 worin Ar, X¹, R¹, R² und m wie in einem der Ansprüche 1 bis 6 definiert sind.

## Claims

1. The use of compounds of the formula I 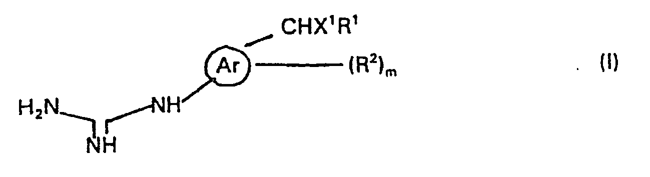 in which
Ar is a benzene ring,
X¹ is a radical of the formula (IIa), (IIb) or (IIc): 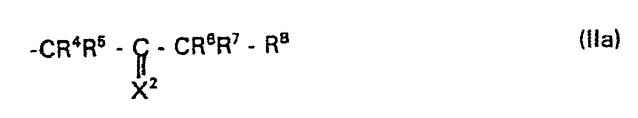 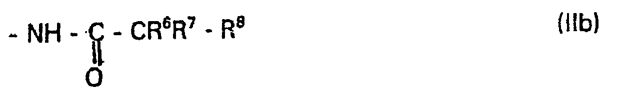 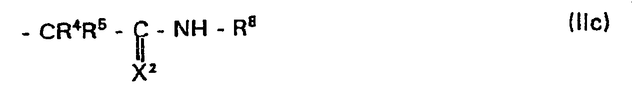
R¹ is H, an unsubstituted or substituted alkyl, alkenyl, alkynyl, aryl or/and heteroaryl radical,
R² is halogen, C(R³)₃), C₂(R³)_{5,} OC(R³)₃ or OC₂(R³)₅,
R³ is in each case independently H or halogen, in particular F,
R⁴ and R⁵ are H or an unsubstituted or substituted alkyl, alkenyl or alkynyl radical, where at least one of the radicals R⁴ and R⁵ contain a hydrogen bond donor group, where the hydrogen bond donor group provides a hydrogen atom or/and an electron pair for a hydrogen bond
R⁶ and R⁷ are H or an unsubstituted or substituted alkyl, alkenyl or alkynyl radical, where at least one of the radicals R⁶ and R⁷ contains a hydrogen bond donor group, where the hydrogen bond donor group provides a hydrogen atom or/and an electron pair for a hydrogen bond and where R⁴ or R⁵ may be bridged with R⁶ or R⁷,
R⁸ is H or an unsubstituted or substituted alkyl, alkenyl, alkynyl, aryl or/and heteroaryl radical or -SO₂-R⁹, where R⁸ may or may not be bridged with R⁶ or R⁷,
R⁹ is H or an unsubstituted or substituted alkyl, alkenyl, alkynyl, aryl or/and heteroaryl radical,
x² is a hydrogen bond acceptor group, in particular NH, NR¹⁰, O or S,
R¹⁰ is an unsubstituted or substituted alkyl, alkenyl or alkynyl radical, and
m is an integer from 0 to 4,
or salts of said compounds for preparing an agent for inhibition of the urokinase plasminogen activator.

2. The use of compounds as claimed in claim 1, in which the substituents -CHX¹R¹ and -NHC(NH)NH₂ are arranged in para position in relation to one another.

3. The use as claimed in either of claims 1 and 2, in which for R⁴, R⁵, R⁶ and R⁷, the hydrogen bond donor groups are selected from OH, OR¹, NH₂, NHR¹, N(R¹)₂, SH and SR¹.

4. The use as claimed in claim 3, in which the substances containing hydrogen bond donor groups are selected from -CH₃OH, -CH₂CH₂OH, -CO₂H, -CO₂R¹, CONH₂, -CONHR¹, -CON(R¹¹)₂, -COSH, -COSR¹, -CSOH, COSR¹, COR¹ and CSR¹, where R¹ is as defined in claim 1.

5. The use as claimed in any of claims 1 to 4, in which for X² the hydrogen bond acceptor groups are selected from O and NH.

6. The use of compounds as claimed in any of claims 1 to 5, in which R⁸ and R⁹ are selected from the group comprising unsubstituted or substituted aryl, in particular phenyl and substituted phenyl, radicals and unsubstituted or substituted tertiary alkyl radicals or cycloalkyl radicals, in particular bicycloalkyl radicals such as adamantyl.

7. The use as claimed in any of claims 1 to 6 for controlling disorders which are associated with a pathological overexpression of urokinase or/and urokinase receptor.

8. The use as claimed in claim 7 for controlling tumors.

9. The use as claimed in claim 7 or 8 for controlling the formation of metastases.

10. The use as claimed in any of the preceding claims for preparing orally, topically, rectally or parenterally administrable medicaments.

11. The use as claimed in any of the preceding claims in the form of tablets, coated tablets, capsules, pellets, suppositories, solutions or transdermal systems such as plasters.

12. Compounds of the formula (I) 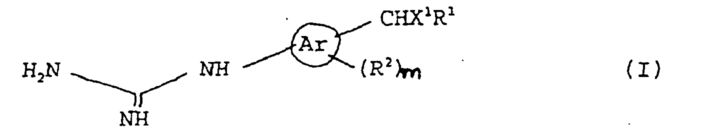 in which Ar, x¹, R¹, R² and m are as defined in any of claims 1 to 6.

## Revendications

1. Utilisation de composés de formule générale (I) 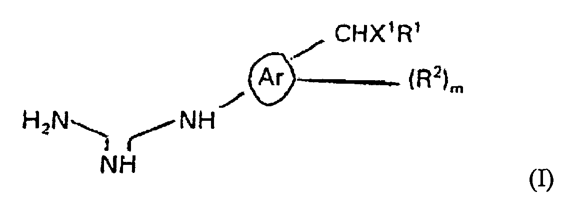 dans laquelle
Ar représente un cycle benzénique,
X¹ représente un groupe de formule (IIa), (IIb) ou (IIc) : 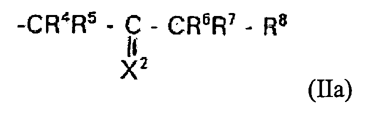 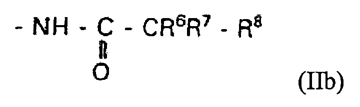 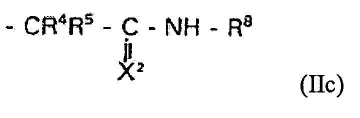
R¹ représente H, un groupe alkyle, alcényle, alcynyle, aryle et/ou hétéroaryle éventuellement substitués,
R² représente halogène, C(R³)₃, C₂(R³)₅, OC(R³)₃ ou OC₂(R³)₅,
R³ représente le cas échéant indépendamment H ou halogène, en particulier F,
R⁴ et R⁵ représentent H ou un groupe alkyle, alcényle ou alcynyle éventuellement substitués, sachant qu'au moins un des groupes R⁴ et R⁵ contient un groupe donneur de liaisons hydrogène, dans laquelle le groupe donneur de liaisons hydrogène apporte un atome H et/ou un doublet d'électrons destinés à une liaison hydrogène,
R⁶ et R⁷ représentent H ou un groupe alkyle, alcényle ou alcynyl éventuellement substitués, sachant qu'au moins un des groupes R⁶ et R⁷ contient un groupe donneur de liaisons hydrogène, dans laquelle le groupe donneur de liaisons hydrogène apporte un atome H et/ou un doublet d'électrons destinés à une liaison hydrogène, et dans laquelle R⁴ ou R⁵ peut être ponté avec R⁶ ou R⁷,
R⁸ représente H ou un groupe alkyle, alcényle, alcynyle, aryle et/ou hétéroaryle ou -SO₂-R⁹ éventuellement substitués, sachant que R⁸ peut être ponté avec R⁶ ou R⁷,
R⁹ représente H ou un groupe alkyle, alcényle, alcynyle, aryle et/ou hétéroaryle éventuellement substitués,
X² représente un groupe accepteur de liaisons hydrogène, en particulier NH, NR¹⁰, O ou S,
R¹⁰ représente un groupe alkyle, alcényle ou alcynyle éventuellement substitués et
m est un nombre entier de 0 à 4,
ou des sels de ces composés pour la fabrication d'un agent destiné à l'inhibition de l'activateur du plasminogène de type urokinase.

2. Utilisation de composés selon la revendication 1, dans laquelle les substituants -CHX¹R¹ et -NHC(NH)NH₂ sont disposés en position para l'un par rapport à l'autre.

3. Utilisation selon l'une quelconque des revendications 1 à 2, dans laquelle les groupes donneurs de liaisons hydrogène des R⁴, R⁵, R⁶ et R⁷ sont choisis parmi OH, OR¹, NH₂, NHR¹, N(R¹)₂, SH et SR¹.

4. Utilisation selon la revendication 3, dans laquelle les substances contenant des groupes donneurs de liaisons hydrogène sont choisies parmi -CH₃OH, -CH₂CH₂OH, -CO₂H, -CO₂R¹, -CONH₂, -CONHR¹, -CON(R¹¹)₂, -COSH, -COSR¹, -CSOH, -COSR¹, COR¹, et CSR¹, sachant que R¹ est défini comme dans la revendication 1.

5. Utilisation selon l'une quelconque des revendications 1 à 4, dans laquelle les groupes accepteurs de liaisons hydrogène X² sont choisis parmi O et NH.

6. Utilisation de composés selon l'une quelconque des revendications 1 à 5, dans laquelle R⁸ et R⁹ sont choisis parmi des groupes aryle éventuellement substitués, en particulier phényle et phényle substitué, et des groupes alkyle tertiaire ou des groupes cycloalkyle éventuellement substitués, en particulier des groupes dicycloalkyle comme l'adamantyle.

7. Utilisation selon l'une quelconque des revendications 1 à 6 destinée à la lutte contre des maladies qui sont associées à une sur-expression pathologique de l'urokinase et/ou du récepteur de l'urokinase.

8. Utilisation selon la revendication 7 destinée à la lutte contre les tumeurs.

9. Utilisation selon la revendication 7 ou 8 destinée à la lutte contre la formation de métastases.

10. Utilisation selon l'une quelconque des revendications précédentes pour la fabrication de médicaments pouvant être administrés oralement, topiquement, par voie rectale ou parentérale.

11. Utilisation selon l'une quelconque des revendications précédentes sous la forme de comprimés, de dragées, de gélules, de pastilles, de suppositoires, de solutions ou de systèmes transdermiques tels que des pansements.

12. Composés de formule (I) 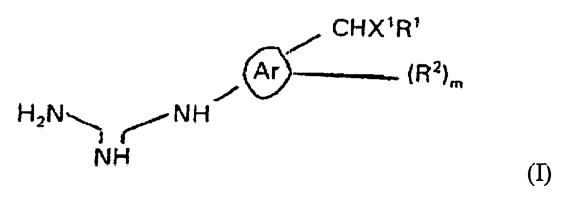 dans laquelle Ar, X¹, R¹, R² et m sont définis comme dans l'une quelconque des revendications 1 à 6.
